Europäisches Patentamt

European Patent Office

Office européen des brevets

(19)

(11) Veröffentlichungsnummer : **0 163 137**
**B1**

(12) # EUROPÄISCHE PATENTSCHRIFT

(45) Veröffentlichungstag der Patentschrift :
02.03.88

(51) Int. Cl.⁴ : **H 04 R 25/00, A 61 F 11/00**

(21) Anmeldenummer : **85105069.0**

(22) Anmeldetag : **26.04.85**

(54) Mehrfrequenz-Übertragungssystem für implantierte Hörprothesen.

(30) Priorität : 30.05.84 DE 3420244

(43) Veröffentlichungstag der Anmeldung :
04.12.85 Patentblatt 85/49

(45) Bekanntmachung des Hinweises auf die Patenterteilung : 02.03.88 Patentblatt 88/09

(84) Benannte Vertragsstaaten :
**AT BE CH FR GB LI**

(56) Entgegenhaltungen :
**EP-A- 0 076 069**
**WO-A-82 /007 60**
**FR-A- 2 383 657**
**US-A- 4 063 048**
**ELEKTROR, Zeitschrift für Elektronik, Nr. 132, Heft 12/81, Dezember 1981, "Hoffnung für Taube", Seiten 12-28, 12-31; Seiten 12-30, Zeilen 37-40**
**SPEKTRUM DER WISSENSCHAFT, April 1985, GERALD E. LOB:"Innenohr-Prothesen", Seiten 104-110, Bild 4,5**

(73) Patentinhaber : **Hortmann GmbH**
**6, Robert-Bosch-Strasse**
**D-7449 Neckartenzlingen (DE)**

(72) Erfinder : **Hortmann, Günter**
**Robert-Bosch-Strasse 6**
**D-7449 Neckartenzlingen (DE)**
Erfinder : **Kunick, Klaus**
**Löwentorstrasse 14**
**D-7000 Stuttgart (DE)**

(74) Vertreter : **Möbus, Rudolf, Dipl.-Ing.**
**Hindenburgstrasse 65**
**D-7410 Reutlingen (DE)**

EP 0 163 137 B1

## Beschreibung

Die Erfindung betrifft ein Mehrfrequenz-Übertragungssystem für implantierte Hörprothesen mit mehreren Reizelektroden, mit einem elektroakustischen Wandler mit Bandpaßfiltern zum Ausfiltern mehrerer Frequenzbänder innerhalb des Hörbereichs, mit einem jedem Frequenzbandkanal zugeordneten Hochfrequenzsender zur Erzeugung eines amplitudenmodulierbaren Hochfrequenz-Trägersignales und mit einem implantierbaren Hochfrequenz-Empfänger mit einem Empfangskanal und einer Reizelektrode für jeden sendeseitigen Frequenzbandkanal.

Zur künstlichen elektrischen Reizung des Innenohres total ertaubter Patienten, deren Hörnerv noch intakt ist, ist es bekannt, eine Mono- oder Multielektrode in die Cochlea einzusetzen, auf welcher mehrere Kontaktkörper verteilt angeordnet sind, die über einen Empfänger durch ein Hochfrequenzsignal von außen erregbar sind und dem Patienten über den Hörnerv einen akustischen Eindruck vermitteln können (siehe WO-Al-82/00760 und EP-Al-0 076 069). Bei solchen Prothesen bestehen die Hauptprobleme darin, die Reizung des Hörnervs des Patienten möglichst naturgetreu zu gestalten, so wie sie auch bei der Schallumwandlung im Innenohr erfolgen würde. Dabei muß vermieden werden, daß durch die mehreren und jeweils einem bestimmten Frequenzspektrum zugeordneten Reizelektroden eine Reizüberlappung auftritt, die es dem Patienten unmöglich machen würde, unterschiedliche Töne voneinander zu unterscheiden. Ein weiteres Problem besteht darin, daß der Hochfrequenz-Empfänger voll implantierbar sein muß, da durch die Haut nach außen ragende Schäfte von Elektrodensockeln oder andere Kontaktverbindungen eine künstliche Hautöffnung erforderlich machen würden, die naturgemäß eine Gefahrenquelle für Infektionen wäre.

Der Erfindung liegt die Aufgabe zugrunde, ein drahtloses Mehrfrequenz-Übertragungssystem für den genannten Zweck so auszubilden, daß es betriebssicherer und mit einer naturgetreueren Hörwirkung als bei bisher bekannten einschlägigen Verfahren und Einrichtungen arbeitet und der erforderliche Energieaufwand niedrig gehalten werden kann.

Die gestellte Aufgabe wird mit einem Mehrfrequenz-Übertragungssystem der eingangs genannten Art erfindungsgemäß dadurch gelöst, daß dem elektroakustischen Wandler eine Sprachprozessorschaltung zugeordnet ist, in welcher hinter jedem Bandpaßfilter ein pegeleinstellbarer Schwellwertschalter für den zugeordneten Hochfrequenz-Generator angeordnet ist, und daß eine Bipolarstufe mit zwei zusätzlichen Hochfrequenz-Generatoren, die abwechselnd Impulse gleicher Länge aber unterschiedlicher Polarität liefern und denen auf der Empfängerseite zwei zusätzliche Empfangskanäle zugeordnet sind, und eine Selektierstufe, die immer nur gleichzeitig das Signal eines der Frequenzbandkanäle und einen Impuls der Bipolarstufe in einer Sendespule am senderseitigen Ausgang auftreten läßt, vorgesehen sind. Das System kann beispielsweise zehn Übertragungskanäle, einschließlich der Übertragungskanäle der beiden zusätzlichen Hochfrequenz-Generatoren der Bipolarstufe, aufweisen.

Das erfindungsgemäß ausgebildete Mehrfachfrequenz-Übertragungssystem hat den Vorteil, daß es einen passiven implantierbaren Empfangsteil aufweist, also keine Energiequelle miteingesetzt werden muß. Bei dem Übertragungssystem müssen die Reizelektroden nicht in die Cochlea eingesetzt werden, sondern können im Mittelohr im Bereich des Promontoriums implantiert werden ; wobei die einzelnen Reizelektroden über den Knochen den Hörnerv erregen. Dies ist erfindungsgemäß möglich, weil sichergestellt ist, daß gleichzeitig immer nur eine Reizelektrode einen bipolaren Reizimpuls liefert, wobei die Bipolarität durch die beiden zusätzlichen Hochfrequenz-Generatoren mit ihren abwechselnden Impulsen gleicher Länge und unterschiedlicher Polarität gesteuert wird. Die Bipolarität der Reizimpulse ist zur Vermeidung elektrolytischer Prozesse bei biologischen Substraten erforderlich. Das Umschalten wird empfängerseitig erfindungsgemäß mittels Schalttransistoren energiearm durchgeführt. Hierzu können die empfängerseitigen Schwingkreise für die Bipolarimpulse jeweils über einen Widerstand mit der Basis eines Schalttransistors verbunden sein, deren Emitter miteinander und deren Kollektoren über Gleichrichter mit allen anderen empfängerseitigen Schwingkreisen verbunden sind.

Ein weiterer wesentlicher und eine Verminderung der Übertragungsenergie bewirkender Vorteil ist bei dem erfindungsgemäß ausgebildeten Mehrfrequenz-Übertragungssystem dadurch gegeben, daß die empfängerseitigen Schwingkreise eine von der dort einstellbaren Lastimpedanz unterschiedliche und hohe Kreisimpedanz aufweisen, so daß nur geringe Schwingkreisströme fließen. Zweckmäßig kann die Sendespule an einem hinter dem Ohr anordenbaren Halter so verstellbar angeordnet sein, daß sie genau über der implantierten Platte dicht gegen den Kopf des Benutzers anlegbar ist, so daß Übertragungsenergieverluste auf ein Minimum reduziert werden.

Das Übertragungssystem gemäß der Erfindung ergibt für den Patienten gute Empfangseigenschaften und größtmögliche Sicherheit, weil einerseits die Ausgangsimpedanz der einzelnen Frequenzbandkanäle und damit die Elektrodenwirkung exakt auf die Hörnervempfindlichkeit abgestimmt werden kann und weil anderseits stets zwei elektromagnetische Hochfrequenzfelder mit den entsprechenden Frequenzen und auch entsprechenden Feldstärken vorhanden sein müssen, um überhaupt eine Reizung zu bewirken. Senderseitig werden Ausgangssignale auf den einzelnen Frequenzbandkanälen nur erzeugt, wenn im betreffenden Hörbereich das auftretende Schaltsignal einen Mindestpegel übersteigt, und das erzeugte Ausgangssignal ist in bekannter Weise entsprechend dem auftretenden Schaltsignal amplitudenmoduliert.

Nachfolgend wird ein Ausführungsbeispiel eines erfindungsgemäß ausgebildeten Mehrfach-Übertragungssystems für implantierte Hörprothesen anhand der beiliegenden Zeichnung näher erläutert.

Im einzelnen zeigen :

Fig. 1 eine Blockschaltdarstellung der Senderseite und der Übertragungsstelle des Übertragungssystems ;

Fig. 2 den Schaltungsaufbau der Empfängerseite ;

Fig. 3 den implantierbaren Reizelektrodenteil der Empfängerseite des Übertragungssystems ;

Fig. 4 einen Schnitt durch den Reizelektrodenteil entlang der Linie IV-IV in Fig.-3 ;

Fig. 5 eine Seitenansicht eines Sendespulenhalters ;

Fig. 6 eine Ansicht des Sendespulenhalters in Richtung des Pfeiles VI in Fig. 5.

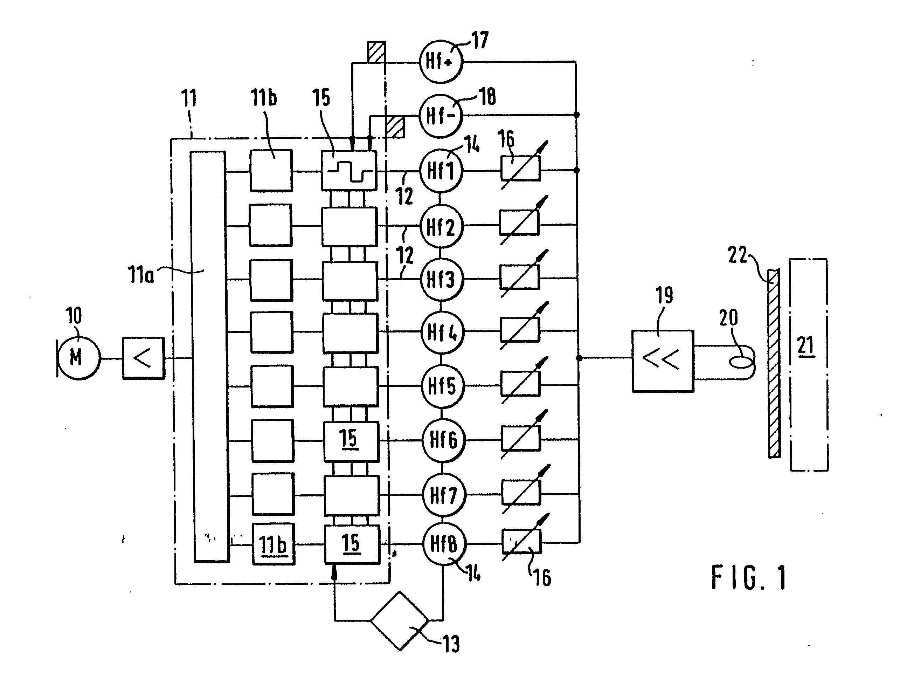

Das schematische Blockschaltbild der Fig. 1 zeigt auf der Senderseite ein Mikrofon 10 als elektroakustischen Wandler, das seine Analogsignale auf einen Sprachprozessor 11 liefert. Im Sprachprozessor 11 wird das elektrische Analogsignal über eine Filterbank 11a in beispielsweise acht Frequenzbänder zerlegt. Der Sprachprozessor 11 umfaßt für jedes Frequenzband einen einstellbaren Schwellwertschalter 11b, wobei die Einstellung beispielsweise 45 bis 70 dB als Schwellwert betragen kann. Erst wenn dieser Schwellwert überschritten wird, wird der dem Frequenzband zugehörige Ausgangskanal 12 aktiviert. Eine dem Sprachprozessor 11 angegliederte Selektierstufe 13 sorgt jedoch dafür, daß immer nur einer der Ausgänge 12 gleichzeitig aktiviert sein kann. Jeder Ausgang 12 führt zu einem Hochfrequenz-Generator 14. Zwischen jedem Hochfrequenz-Generator 14 und dem Schwellwertschalter 11b ist eine Impulsformerstufe 15 angeordnet. Jeder der Hochfrequenz-Generatoren 14 liefert ein Trägerfrequenzsignal mit einer bestimmten Frequenz, die im Gesamtfrequenzbereich zwischen 1 und 5 MHz liegt. Dieses Trägersignal kann entsprechend dem empfangenen Schaltsignal amplitudenmoduliert werden. Den Hochfrequenz-Generatoren 14 nachgeschaltete Potentiometer 16 erlauben eine Einstellung der Signalamplitude jedes einzelnen Kanals, an eine unterschiedliche Reizempfindlichkeit der einzelnen Elektroden der Empfängerseite.

Der Sprachprozessor 11 ist außerdem mit zwei weiteren Hochfrequenz-Generatoren 17 und 18 verbunden, von denen der eine Hochfrequenz-Generator 17 durch positive und der andere Hochfrequenz-Generator 18 durch negative Impulse eingeschaltet wird, die zeitlich aufeinanderfolgen und aus Symmetriegründen gleiche Länge haben. An der gemeinsamen Hochfrequenz-Endstufe 19 des Übertragungssystems tritt somit immer ein Signal von abwechselnd dem Hochfrequenz-Generator 17 und dem Hochfrequenz-Generator 18 zusammen mit einem Signal eines einzigen der acht Hochfrequenz-Generatoren 14 auf, und diese kombinierten Signale werden über eine Sendespule 20, die hinter dem Ohr eines Patienten anbringbar ist, auf einen ebenfalls hinter dem Ohr des Patienten implantierten Empfänger 21 übertragen, also drahtlos durch die Haut 22 des Patienten hindurch.

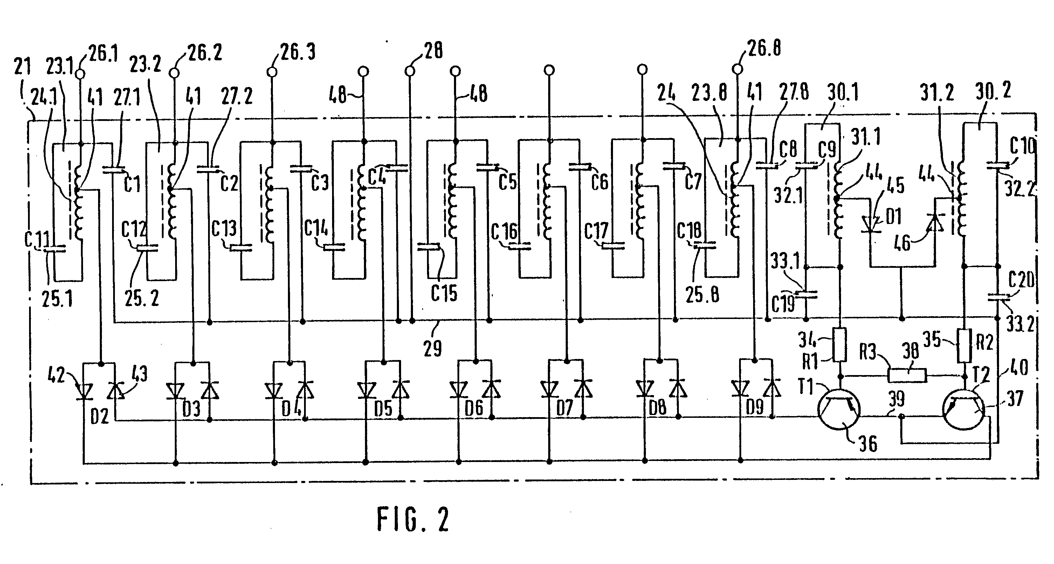

Fig. 2 zeigt den Schaltungsaufbau des implantierbaren Empfängers 21. Der Empfänger weist für jeden Frequenzbandkanal einen Schwingkreis 23.1... 23.8 mit einer Empfangsspule 24.1... 24.8 auf. Die Empfangsspule 24.1-24.8 wirkt jeweils mit einem Schwingkreiskondensator 25.1... 25.8 zusammen. An jeden Schwingkreis ist eine Leitung 26.1, 26.2... 26.8 zu einer Reizelektrode angeschlossen, und jeder Schwingkreisausgang ist über einen Kondensator 27.1... 27.8 mit einem Anschluß 28 zu einer Neutralelektrode gekoppelt. Die gemeinsame Verbindungsleitung zum Neutralelektrodenanschluß 28 ist durch die Bezugsziffer 29 gekennzeichnet.

Der Empfänger weist außerdem zwei Schwingkreise 30.1 und 30.2 für die Signale der beiden zusätzlichen Hochfrequenz-Generatoren 17 und 18 der Bipolarstufe der Sendeseite auf. Die Schwingkreise bestehen aus den Empfangsspulen 31.1 und 31.2 sowie Kondensatoren 32.1 und 32.2. Auch die beiden Schwingkreise 30.1 und 30.2 sind über Ladekondensatoren 33.1 und 33.2 mit der Neutralelektrode 28 verbunden. Außerdem sind die Ausgänge der beiden Schwingkreise 30.1 und 30.2 über Widerstände 34 oder 35 mit der Basis von zwei gegensinnig wirkenden Transistoren 36 und 37 verbunden, wobei zwischen den Basen der beiden Transistoren 36 und 37 noch ein Koppelungswiderstand 38 vorgesehen ist. Die Emitter der beiden Transistoren 36 und 37 sind durch eine Leitung 39 miteinander und über eine Leitung 40 und die gemeinsame Leitung 29 mit der Neutralelektrode 28 verbunden.

Jede Empfangsspule 24.1-24.8 ist mit einem Abgriff 41 versehen, der jeweils über einen von zwei gegensinnig geschalteten Gleichrichtern 42 und 43 mit dem Kollektor des Transistors 36 bzw. dem Kollektor des Transistors 37 verbunden ist. Auch die beiden Empfangsspulen 31.1 und 31.2 sind mit einem Abgriff 44 versehen, der über einen Gleichrichter 45 oder 46 mit der gemeinsamen Verbindungsleitung 29 verbunden ist.

Durch die mit Abgriffen 41 und 44 versehenen Empfangsspulen 24.1-24.8 und 31.1, 31.2 wird erreicht, daß die Impedanz der Schwingkreise 23.1-23.8, 30.1, 30.2 größer ist als die Ausgangsimpedanz, in den Schwingkreisschaltungen also nur kleine Kreisströme auftreten.

Wie bereits erwähnt, erfolgt das Einschalten der Hochfrequenz-Generatoren 14 der Senderseite durch vom Sprachprozessor 11 erzeugte Impulse. Gleichzeitig wird je nach Polarität des Sprachprozessorimpulses einer der beiden zusätzlichen Hochfrequenz-Generatoren 17, 18 eingeschaltet, welcher im Empfänger 21 den Transistor 36 oder 37 leitend macht und somit eine positive oder negative Gleichrichtung des auf den Empfänger übertragenen Signales bewirkt. Ändert der Steuerimpuls des Sprachprozessors seine Polarität, so schaltet der erste zusätzliche Hochfrequenz-Generator 17 ab und der zweite zusätzliche Hochfrequenz-Generator 18 wird eingeschaltet, so daß im Empfänger eine Umkehr der Gleichrichtungspolarität bewirkt wird: Der Energieverbrauch im Empfänger ist durch die gewählte Schaltung gering. Aktive Bauelemente mit einem hohen Energieeigenverbrauch sind vermieden.

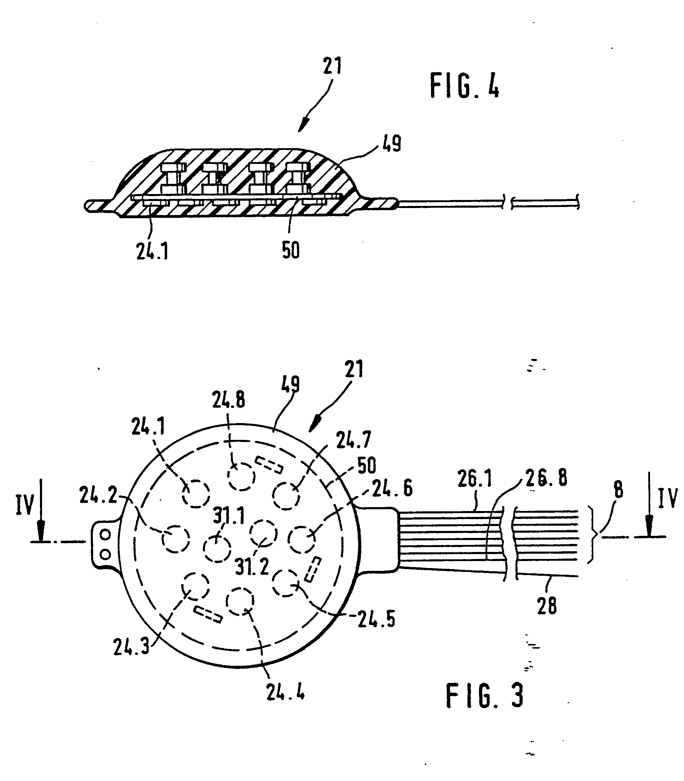

Die Fig. 3 und 4 zeigen den räumlichen Aufbau des implantierten Empfängers 21. Auf einem dünnen, beidseitig kupferbeschichteten Plattenkörper 50 sind auf der einen Seite die insgesamt zehn Empfangsspulen 24.1-24.8 und 31.1, 31.2 dicht beieinander auf einem Durchmesser von nur 25 mm angeordnet. Auf der anderen Seite befinden sich im wesentlichen die übrigen elektronischen Bauelemente, welche mit Hilfe von auf den Plattenkörper beiderseits geätzten Leiterbahnen miteinander verbunden sind.

Zur Vermeidung des Eindringens von Körperflüssigkeiten wird der gesamte elektronische Aufbau in einen körperverträglichen Kunststoff entsprechend der gestrichelten Kontur 49 eingegossen.

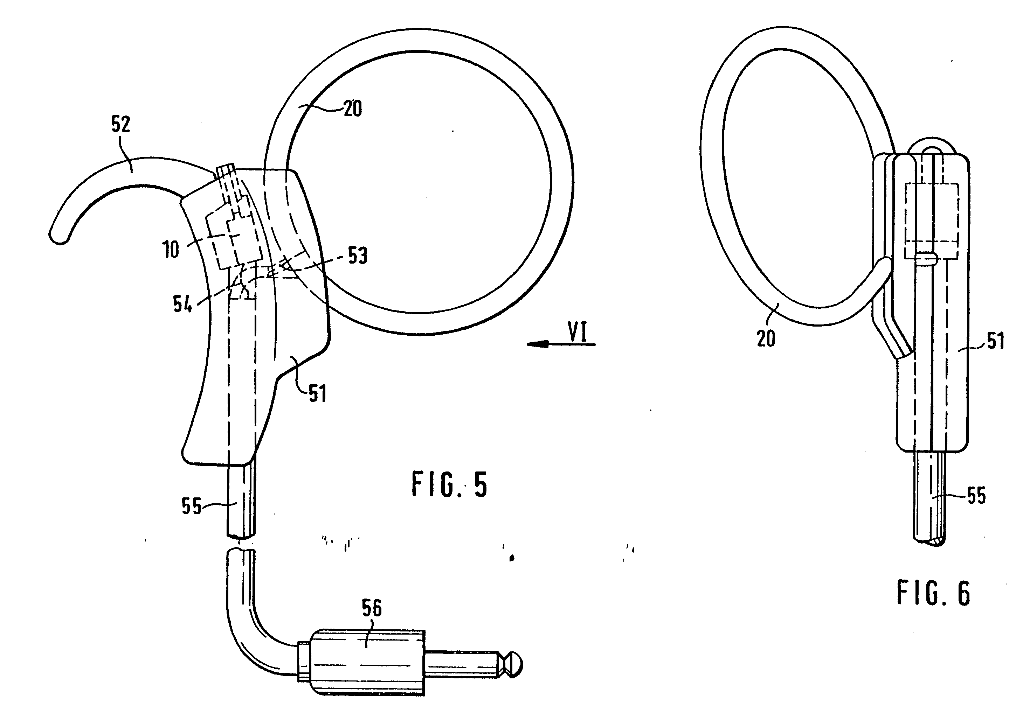

Die Fig. 5 und 6 zeigen einen hornförmigen Halter 51, in welchem das Mikrofon 10 des Übertragungssystemes hinter einer nicht näher dargestellten Schallaufnahmeöffnung angeordnet ist und in welchem die Sendespule 20 aus der Ebene des hornförmigen Trägers verschwenkbar gelagert ist. Fig. 6 zeigt die Sendespule 20 beispielsweise unter einem Winkel von ca. 30° zur Ebene des hornförmigen Trägers 51 verschwenkt. Der Träger 51 ist mit einem anpaßbaren Bügel 52 versehen, mit welchem er am Ohr eines Patienten so eingehängt werden kann, daß der Halter 51 hinter dem Ohr liegt und die Sendespule 20 dicht am Kopf und genau über dem implantierten Empfänger 21, also der Implantierten Platte 50, zu liegen kommt. Die Anschlußleitung 53 der Sendespule 20 und die Anschlußleitung 54 des Mikrofons 10 sind in einem gemeinsamen Kabel 55 aus dem Halter 51 herausgeführt. Das Kabel 55 endet in einem Stecker 56 zur Verbindung mit den übrigen Teilen der Senderseite des Mehrfrequenz-Übertragungssystemes, die vom Patienten an einer passenden Stelle getragen werden.

In dem Halter 51 können weitere elektrische Teile der Senderseite des Übertragungssystems untergeordnet sein. Das Mikrofon 10 sitzt also dicht bei der Stelle, wo bei einem gesunden Ohr die Schallwellen aufgenommen werden, und der Halter erlaubt eine individuelle Anpassung der Lage der Sendespule am Kopf des Patienten in Ausrichtung auf die bei ihm implantierte Platte 50.

## Patentansprüche

1. Mehrfrequenz-Übertragungssystem für implantierte Hörprothesen mit mehreren Reizelektroden, mit einem elektroakustischen Wandler (10) mit Bandpaßfiltern (11a) zum Ausfiltern mehrerer Frequenzbänder innerhalb des Hörbereichs, mit einem jedem Frequenzbandkanal zugeordneten Hochfrequenzsender (14) zur Erzeugung eines amplitudenmodulierbaren Hochfrequenz-Trägersignales und mit einem implantierbaren Hochfrequenz-Empfänger (21) mit einem Empfangskanal und einer Reizelektrode für jeden sendeseitigen Frequenzbandkanal, dadurch gekennzeichnet, daß dem elektroakustischen Wandler (10) eine Sprachprozessorschaltung (11) zugeordnet ist, in welcher hinter jedem Bandpaßfilter ein pegeleinstellbarer Schwellwertschalter (11b) für einen Hochfrequenz-Generator (14) angeordnet ist, und daß eine Bipolarstufe mit zwei zusätzlichen Hochfrequenz-Generatoren (17, 18), die abwechselnd Impulse gleicher Länge aber unterschiedlicher Polarität liefern und denen auf der Empfängerseite (21) zwei zusätzliche Empfangskanäle (30.1/30.2) zugeordnet sind, und eine Selektierstufe (13), die immer nur gleichzeitig das Signal eines der Frequenzbandkanäle und einen Impuls der Bipolarstufe in einer Sendespule (20) am senderseitigen Ausgang auftreten läßt, vorgesehen sind.

2. Mehrfrequenz-Übertragungssystem nach Anspruch 1, dadurch gekennzeichnet, daß der Empfänger (21) für jedes übertragene Frequenzbandsignal und für beide Bipolarsignale einen Schwingkreis (23.1-23.8 ; 30.1, 30.2) mit von der einstellbaren Lastimpedanz unterschiedlicher und hoher Kreisimpedanz aufweist.

3. Mehrfrequenz-Übertragungssystem nach Anspruch 1 oder 2, dadurch gekennzeichnet, daß die empfängerseitigen Schwingkreise (30.1, 30.2) für die Bipolarimpulse jeweils über einen Widerstand (34, 35) mit der Basis eines Schalttransistors (36, 37) verbunden sind, deren Emitter miteinander und deren Kollektoren über Gleichrichter (42, 43) mit allen anderen empfängerseitigen Schwingkreisen (23.1-23.8) verbunden sind.

4. Mehrfrequenz-Übertragungssystem nach einem der Ansprüche 1 bis 3, dadurch gekennzeichnet, daß die Frequenzbänder innerhalb eines Gesamtfrequenzbereiches von 1-5 MHz liegen.

5. Mehrfrequenz-Übertragungssystem nach einem der Ansprüche 1 bis 4, dadurch gekennzeichnet, daß der Empfänger (21) mit zehn Schwingkreisen (23.1-23.8, 30.1, 30.2) und mit den Anschlüssen der Reizelektroden (26.1-26.8) auf einer hinter dem Ohr implantierbaren Platte (50) von maximal 25 mm Durchmesser aufgebracht ist.

6. Mehrfrequenz-Übertragungssystem nach einem der Ansprüche 1 bis 5, dadurch gekennzeichnet, daß die Sendespule (20) an einem Halter (51) befestigt ist, der den elektroakustischen Wandler (10) trägt und mit einem anpaßbaren Bügel (52) zum Einhängen sm Ohr versehen ist, dergestalt, daß der Halter (51) hinter dem Ohr mit der Sendespule (20) über der implantierten Platte (50) zu liegen kommt.

7. Mehrfrequenz-Übertragungssystem nach Anspruch 6, dadurch gekennzeichnet, daß die Sendespule (20) am Halter (51) zur dichten Anlage gegen den Kopf aus der Halterebene verschwenkbar angeordnet ist.

8. Mehrfrequenz-Übertragungssystem nach Anspruch 6 und 7, dadurch gekennzeichnet, daß der elektroakustische Wandler (10) und die Sendespule (20) über ein mehradriges Kabel (56) mit dem vom Benutzer an passender Stelle getragenen übrigen Sendeteil verbunden sind.

## Claims

1. Multifrequency transmission system for implanted hearing aids having several stimulating electrodes, comprising and electro-acoustic transducer (10) with band-pass filters (11a) for filtering out several frequency bands within the audible range, a high-frequency transmitter (14), associated with each frequency-band channel, for the generation of an amplitude-modulable high-frequency carrier signal and an implantable high-frequency receiver (21) with a reception channel and a stimulating electrode for each transmission-side frequency-band channel, characterised in that associated with the electro-acoustic transducer (10) is a speech processor circuit (11) in which a level-adjustable threshold switch (11b) for a high-frequency generator (14) is arranged behind each band-pass filter, and in that a bipolar stage with two additional high-frequency generators (17, 18), which alternately supply impulses of the same length but of different polarity and associated with which on the receiver side (21) are two additional reception channels (30.1/30.2), and a selecting stage (13) which always allows the signal of one of the frequency-band channels and a pulse of the bipolar stage to occur only simultaneously in a transmission coil (20) at the transmitter-side output, are provided.

2. Multifrequency transmission system according to claim 1, characterised in that the receiver (21) for each transmitted frequency-band signal and for both bipolar signals has an oscillatory circuit (23.1- 23.8 ; 30.1, 30.2) with a high circuit impedance which is different from the adjustable load impedance.

3. Multifrequency transmission system according to claim 1 or 2, characterised in that the receiver-side oscillatory circuits (30.1, 30.2) for the bipolar pulses are in each case connected by way of a resistor (34, 35) to the base of a switching transistor (36, 37), the emitters of which are connected to one another and the collectors of which are connected by way of rectifiers (42, 43) to all the other receiver-side oscillatory circuits (23.1-23.8).

4. Multifrequency transmission system according to one of claims 1 to 3, characterised in that the frequency bands lie within an overall frequency range of 1-5 MHz.

5. Multifrequency transmission system according to one of claims 1 to 4, characterised in that the receiver (21) with ten oscillatory circuits (23.1-23.8, 30.1, 30.2) and with the connections of the stimulating electrodes (26.1-26.8) is mounted on a plate (50) of a maximum diameter of 25 mm which is implanted behind the ear.

6. Multifrequency transmission system according to one of claims 1 to 5, characterised in that the transmission coil (20) is fastened to a holder (51) which carries the electro-acoustic transducer (10) and is provided with an adaptable stirrup (52) for suspension from the ear, in such a way that the holder (51) comes to rest behind the ear with the transmission coil (20) over the implanted plate (50).

7. Multifrequency transmission system according to claim 6, characterised in that the transmission coil (20) is arranged on the holder (51) so as to be swingable out of the holder plane for tight abutment against the head.

8. Multifrequency transmission system according to claim 6 and 7, characterised in that the electro-acoustic transducer (10) and the transmission coil (20) are connected by way of a multiconductor cable (56) to the remaining transmission part carried by the user at a suitable location.

## Revendications

1. Système de transmission à fréquences multiples pour prothèses auditives implantées à plusieurs électrodes excitatrices, comprenant un transducteur électro-acoustique (10) à filtres passe-bande (11a) pour éliminer par filtrage plusieurs bandes de fréquence à l'intérieur de la plage audible, un émetteur à haute fréquence (14) affecté à chaque canal de bande de fréquence pour produire un signal porteur à haute fréquence à amplitude modulable, et un récepteur à haute fréquence implantable (21) comportant un canal de réception et une électrode excitatrice pour chaque canal de bande de fréquence côté émetteur, caractérisé en ce qu'au transducteur électro-acoustique (10) est affecté un circuit de traitement vocal (11) dans lequel un commutateur à seuil à niveau réglable (11b) pour un générateur à haute fréquence correspondant (14) est disposé derrière chaque filtre passe-bande, et en ce que sont prévus un étage bipolaire à deux générateurs à haute fréquence supplémentaires (17, 18), qui fournissent alternativement des impulsions de même longueur, mais de polarités différentes et auxquels sont affectés deux canaux de réception supplémentaires (30.1/30.2) côté récepteur (21), et un étage de sélection (13) qui ne permet de fournir à la fois que le signal de l'un des canaux de bande de fréquence et une impulsion de l'étage bipolaire dans une bobine émettrice (20) à la sortie côté émetteur.

2. Système de transmission à fréquences multiples conforme à la revendication 1, caractérisé en ce que le récepteur (21) comprend, pour chaque signal de bande de fréquence transmis et pour les deux signaux bipolaires, un circuit oscillant (23.1 à 23.8 ; 30.1, 30.2) à impédance de circuit élevée et différente de l'impédance de charge réglable.

3. Système de transmission à fréquences multiples conforme à la revendication 1 ou 2, caractérisé en ce que les circuits oscillants côté récepteur (30.1, 30.2) destinés aux impulsions bipolaires sont respectivement connectés, par l'intermédiaire de résistances (34, 35), aux bases de transistors de commutation (36, 37) dont les émetteurs sont connectés ensemble et dont les collecteurs sont connectés, par l'intermédiaire de redresseurs (42, 43), à tous les autres circuits oscillants côté récepteur (23.1 à 23.8).

4. Système de transmission à fréquences multiples conforme à l'une des revendications 1 à 3, caractérisé en ce que les bandes de fréquence sont comprises à l'intérieur d'une plage totale de fréquences de 1 à 5 MHz.

5. Système de transmission à fréquences multiples conforme à l'une des revendications 1 à 4, caractérisé en ce que le récepteur (21) comportant dix circuits oscillants (23.1 à 23.8, 30.1, 30.2) et les connexions des électrodes excitatrices (26.1 à 26.8) est supporté par une plaque (50) d'un diamètre maximum de 25 mm implantable derrière l'oreille.

6. Système de transmission à fréquences multiples conforme à l'une des revendications 1 à 5, caractérisé en ce que la bobine émettrice (20) est fixée à un support (51) qui porte le transducteur électro-acoustique (10) et qui est muni d'un étrier adaptable (52) pouvant être accroché à l'oreille, de façon que le support (51) soit appliqué derrière l'oreille, avec la bobine émettrice (20) au-dessus de la plaque implantée (50).

7. Système de transmission à fréquences multiples conforme à la revendication 6, caractérisé en ce que la bobine émettrice (20) est montée sur le support (51) de manière à pouvoir pivoter hors du plan du support, tout contre la tête.

8. Système de transmission à fréquences multiples conforme aux revendications 6 et 7, caractérisé en ce que le transducteur électro-acoustique (10) et la bobine émettrice (20) sont connectés, par l'intermédiaire d'un câble multifilaire (56), au reste de la partie d'émetteur que l'utilisateur porte en un endroit approprié.

FIG. 1

0 163 137

FIG. 2

0 163 137

FIG. 4

FIG. 3

FIG. 6

FIG. 5